# EUROPEAN PATENT SPECIFICATION

(11) **EP 1 020 180 B1**
(45) Date of publication and mention of the grant of the patent: **31.03.2004**
(21) Application number: 00300073.4
(22) Date of filing: 07.01.2000
(51) Int. Cl.: A61K 9/08, A61K 47/02

(54) **Biocompatible, injectable aqueous solution for use in ultrasound energy assisted surgery**
Biokompatible injektierbare wässerige Lösung zur Verwendung bei ultraschallenergieunterstützter Chirurgie
Solution aqueuse injectable et biocompatible pour utilisation en chirurgie assistée par énergie ultrasonique

(30) Priority: 07.01.1999 IL 12796799
(43) Date of publication of application: 19.07.2000
(73) Proprietor: Topaz, Moris, Kibbutz Ramat HaKovesh 44930 (IL)
(72) Inventor: Topaz, Moris, Kibbutz Ramat HaKovesh 44930 (IL)
(74) Representative: Ablewhite, Alan James

(56) References cited:
- WO-A-94/06355
- US-A- 5 149 319
- US-A- 5 582 829
- ML ZOCCHI: "Ultrasonic assisted lipoplasty" CLINICS IN PLASTIC SURGERY, vol. 23, no. 4, October 1996 (1996-10), pages 575-598, XP008003875 USA

## Description

### FIELD OF INVENTION:

The present invention relates to a biocompatible, injectable aqueous solution for use in high intensity ultrasound energy assisted surgery. More specifically the irrigation solution of the present invention contains a gas selected from the group consisting of carbon dioxide, nitrogen and mixtures thereof for the reduction and limitation of cavitation and the reduction and limitation of sonoluminescence and sonochemistry.

### BACKGROUND OF THE INVENTION:

Ultrasound energy has been extensively used in the medical field, mainly for diagnostic and therapeutic purposes. It has been utilized for diagnosis in medicine mainly at a sonar pulse mode of very short duration and of high frequency and peak intensity, or at low intensities of 10⁻² to 10⁻³ W/cm² CW, and at a high frequency in dopler mode. Ultrasound is used in physical therapy in intensities of up to 3-5 W/cm² CW, for a short exposure time. Ultrasonic ablative surgery instrumentation, such as those used in phacoemulsification in ophthalmology, recanalization and thrombus dissolution in peripheral arteries, in neurosurgery, hepatic, renal, prostate and bladder resections, as well as in extracorporeal shock wave lithotripter, utilize higher power intensities that are above 10W/cm²CW. The long-term safety of diagnostic ultrasonic equipment in clinical use was questioned and evaluated with no significant effects demonstrated at intensities below 100 mW/cm² spatial and temporal peak average intensity.

High-intensity ultrasound energy (HIUE) has been used in the last three decades in a wide range of surgical procedures for the extraction of tissues such as soft tissues and other target tissues, said target tissues including kidney tissues, brain tissues, liver tissues, cataracts, etc. In the last ten years, HIUE has been utilized for aesthetic surgery in Ultrasound-Assisted Lipoplasty (UAL). In UAL, subcutaneous fatty tissue in the human body is irrigated with wetting solution, emulsified by exposure to HIUE through a probe via a stab wound in the skin, and can be evacuated simultaneously or immediately following, by the application of low-vacuum suction.

The standard conditions for operating HIUE are usually at an intensity range above continuous wave (CW) 10W/cm², frequencies in the range of about 20-100 kHz, and amplitudes in the range of 60 (low) - 320 (ultrahigh) micrometers, double (peak-to-peak) amplitude of the radiation face of the tip in micrometers on sonicator processor operating at maximum output.

In order to avoid the thermal effect resulting from the exposure of soft tissue to HIUE, a physiological water solution is injected into the subcutaneous tissue before and/or during irradiation, resulting in a substantial reduction in temperature rise, and peak temperature, thus avoiding thermal burn. The application of HIUE in an aqueous medium, however, has been proved to generate cavitation.

Cavitation is the expansion and rapid adiabatic collapse of tiny gas bubbles in solution, with energy sufficient to produce sonoluminescence and sonochemistry and temperature rise. Sonoluminescence is the conversion of sound energy into electromagnetic energy in the form of light in the visible, UV and possible soft x-ray range of the spectrum. Sonoluminescence is the direct indication of cavitation generated by the application of HIUE to aqueous solution. Sonochemistry is the generation of highly reactive chemical products such as free radicals and oxidizing reactants in the cavitation medium.

UV light and free radicals were found in previous scientific reports to lead to changes in biological systems, by alteration of the DNA sequence, changes in RNA, cell membranes, other cell infrastructures and in other macromolecules. A review article by the present inventor that appeared in the Aesthetic Surgery Journal 1998;18:19-24, "Possible Long-term Complications in Ultrasound-assisted Lipoplasty Induced by Sonoluminesence, Sonochemistry, and Thermal Effect" by the present inventor, as well as a letter to the editor "Long-Term Possible Hazardous Effects of Ultrasonically Assisted Lipoplasty" published in Plastic and Reconstructive Surgery, 1998;102:280, sparked controversy regarding possible long-term adverse effects of UAL. In further work, the present inventor has found that continuous wave (CW) HIUE applied under conditions that simulate UAL operating in an intercellular environment and current instrumentation, is above threshold intensities under which large quantities of free radicals and oxydizing reactants are produced, and in which sonoluminescence is generated. This irradiation may induce long-term risks for patients undergoing exposure to HIUE, particularly in long exposure, the exposure of sensitive tissue such as breast and nerve, and in young-age patients.

The use of scavengers in oral administration, such as vitamins A, C, D, E and others, was proposed prior to or after exposure to sonication in order to reduce the possible effects of free radicals. The limitation of the oral application of scavengers lies in the need for high concentrations of these substances to avoid the effects of free radicals. The addition the above substances to the sonicated medium does not inhibit cavitation with the subsequent generation of sonoluminescence and Sonochemistry.

A known method for eliminating cavitation is by degassing of the solution prior to the application of HIUE. The above method would be impractical in a common surgical procedure because gas would infiltrate into the wetting solution through the stab wounds in the skin to the subcutaneous tissue. It would be very hard to prevent gases from entering the tissue and solution through the surgical ports during the steps of injecting wetting solution, elevation or retraction of the skin prior or during sonication. All of the above steps would allow gas to reenter the subcutaneous tissue and the sonicated solution.

The application of HIUE to tissue leads to fragmentation in a complicated mechanism. The mechanical, longitudinal action of the probe creates a shearing effect which leads to a sharp and drastic elevation in pressure and temperature and thus damage or destruction of tissue structures cell membrane, and other infrastructures. The addition of water to the biological environment would actually significantly reduce the rate of temperature elevation and the peak temperature, yet would lead to cavitation that would eventually lead to the formation of sonochemistry and sonoluminescence. As is realized from the above, there exists a need to eliminate cavitation during the sonication of tissue.

According to the invention, there is provided the use of a gas selected from the group consisting of carbon dioxide, nitrogen, and mixtures thereof in the preparation of a biocompatible, injectable aqueous solution of the gas for use in high intensity ultrasound energy assisted surgery at greater than 10 W/cm²CW at 20-100 kHz with an amplitude of 60-320 µm.

In one embodiment of the invention, said high intensity ultrasound energy assisted surgery is ultrasound assisted lipoplasty. This lipoplasty may in particular be for cosmetic treatment. That is, the invention may be used for purely cosmetic purposes.

Preferably, said gas is carbon dioxide. Also, the gas is preferably passed through the solution at a flow rate of from 10 to 500 ml/min, more preferably at a flow rate of from 50 to 200 ml/min, and most preferably at a flow rate of approximately 100 ml/min.

In a preferred aspect of the invention, the biocompatible, injectable aqueous solution for use in the manufacture of a medicament for use in high intensity ultrasound energy assisted surgery comprises a dissolved gas selected from the group consisting of nitrogen, carbon dioxide and mixtures thereof, at a concentration which is higher than the concentration in a solution which is in equilibrium with air at 25°C and one atmosphere pressure.

In a preferred embodiment of the present invention the solution is for use in ultrasound assisted lipoplasty comprising carbon dioxide for the reduction and limitation of cavitation.

The present invention thus enables the use of a gas in the preparation of a biocompatible, injectable aqueous solution of the gas for reducing and limiting cavitation in ultrasound assisted lipoplasty comprising, irrigating an area of target fatty tissue with an irrigating solution, inserting a probe into said area, ultrasonically vibrating said probe at standard conditions creating localized tissue separation and frictional heat; emulsification of at least some of said fatty tissue by said mechanical and sonic vibrations, so as to provide safer removal of said fatty tissue, emulsifying said fatty tissue; and evacuating the emulsified fatty tissue by applying suction, characterized in that said irrigating solution further comprises a gas selected from the group consisting of carbon dioxide, nitrogen and mixtures thereof.

In a preferred embodiment of the present invention there is enabled the use of a gas in the preparation of a biocompatible, injectable aqueous solution of the gas for removing target tissue from a patient comprising: , irrigating an area of target tissue with an irrigating solution, inserting a probe into said area of target tissue, ultrasonically vibrating said probe at standard conditions creating localized tissue separation and frictional heat; emulsifying at least some of said target tissue by said mechanical and sonic vibrations, so as to provide safer removal of said target tissue, emulsifying said target tissue; and evacuating the emulsified target tissue by applying suction, characterized in that a gas selected from the group consisting of carbon dioxide, nitrogen and mixtures thereof is introduced into said irrigating solution in the area exposed to high intensity ultrasound energy.

An even more preferred embodiment of the present invention enables the use of a gas in the preparation of a biocompatible, injectable aqueous solution of the gas for removing fatty tissue from a patient comprising: irrigating an area of target fatty tissue with an irrigating solution, inserting a probe into said area of fatty tissue; ultrasonically vibrating said probe at standard conditions creating localized tissue separation and frictional heat; emulsifying at least some of said fatty tissue by said mechanical and sonic vibrations, so as to provide safer removal of said fatty tissue, emulsifying said fatty tissue; and evacuating the emulsified fatty tissue by applying suction, characterized in that a gas selected from the group consisting of carbon dioxide, nitrogen and mixtures thereof is introduced into said irrigating solution in the area sonicated by said probe.

In especially preferred embodiment there is enabled the use of a gas in the preparation of a biocompatible, injectable aqueous solution of the gas for removing target tissue from a patient wherein said gas is present in an amount sufficient to reduce cavitation in the area of sonication.

In an even further preferred embodiment the use of the present invention further comprises introducing a water soluble scavenger into said irrigating solution wherein said water soluble scavenger is vitamin C or a source thereof.

In the most preferred embodiments of the present invention said gas is carbon dioxide, however as will be realized the carbon dioxide used for suppressing cavitation, sonoluminescence and sonochemistry according to the present invention can be used in combination with other acceptable gases such as nitrogen.

The invention will now be described in connection with certain preferred embodiments with reference to the following illustrative figures so that it may be more fully understood.
Figure 1 is a comparative graphical representation of the influence of various gases on sonoluminescence generated by HIUE.
Figure 2 is a comparative graphical representation of the influence of various gases on chemi-sonoluminescence.
Figure 3 is a comparative graphical representation of the influence of various gases on the formation of hydroxyl free-radicals.

With specific reference now to the figures in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

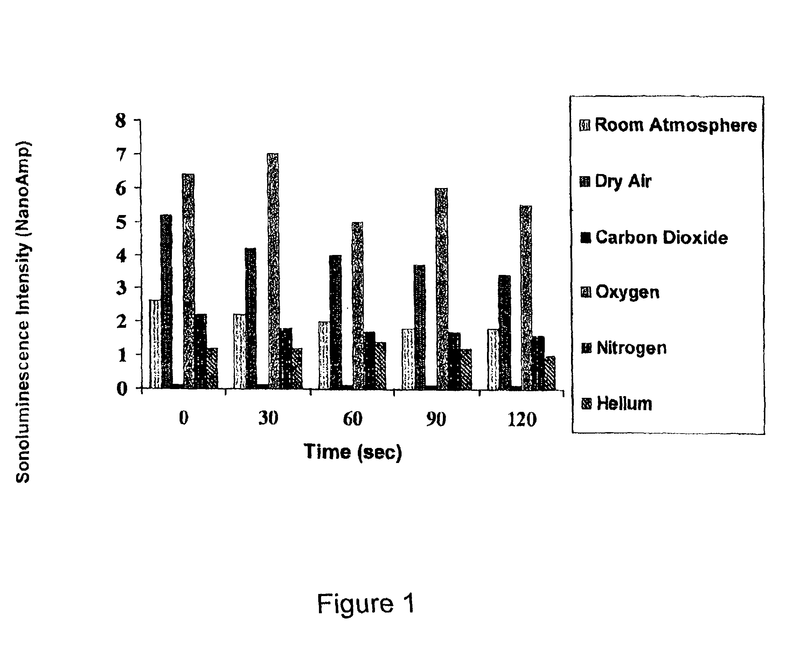

Figure 1 illustrates values of Sonoluminescence that were detected by sensitive photomultiplication technique. Sonoluminescence was generated by the cavitation phenomenon by sonication of aqueous physiological solution with HIUE in the presence of various gases. The gas flow was 100 ml/min in a saturated saline solution in order to determine the effect each gas had on Sonoluminescence. As can be seen the presence of carbon dioxide nearly eliminated Sonoluminescence thereby eliminating the creation of ultra violet light and possible soft x-ray radiation, while the use of nitrogen substantially reduced the same.

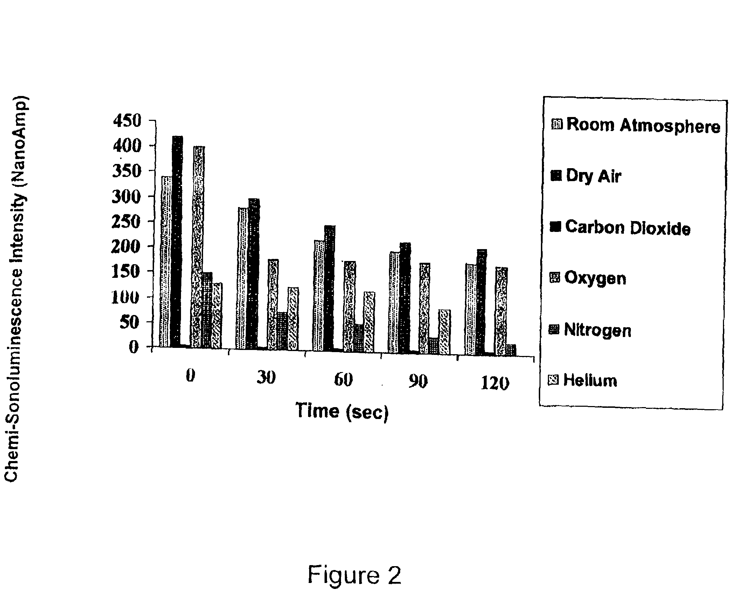

Figure 2 illustrates values of Chemi-Sonoluminescence that were detected by sensitive photomultiplication technique. Chemi-Sonoluminescence was generated by the cavitation phenomenon in presence of Luminol (200 micL 10⁻² M Luminol, 50% Intensity amplitude) by sonication of aqueous physiological solution with HIUE in the presence of various gases. The gas flow was 100 ml/min in a saturated saline solution in order to determine the effect each gas had on Chemi-sonoluminescence. As can be seen the presence of various gases resulted in varying intensities of Chemi-sonoluminescence depending upon the effect of said gases on cavitation.

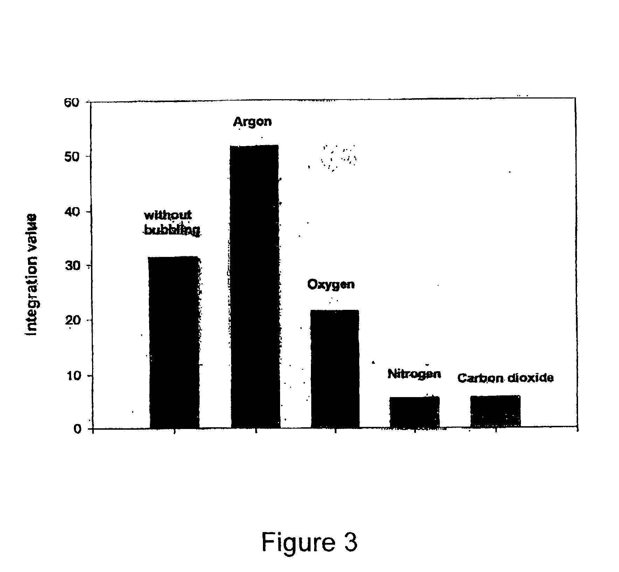

Figure 3 illustrates the relative Integration values of an electron spin resonance (ESR) spectra of the DMPO-OH spin adduct signal peaks generated by sonication of solution which contained saline and each of the gases mentioned within the figure. The sonication conditions were 20sec., 3.4 relative intensity. As can be seen the presence of carbon dioxide significantly reduced the generation of hydroxy free radicals, as did the presence of nitrogen.

## Claims

1. Use of a gas selected from the group consisting of carbon dioxide, nitrogen, and mixtures thereof in the preparation of a biocompatible, injectable aqueous solution of the gas for use in high intensity ultrasound energy assisted surgery at greater than 10 W/cm²CW at 20-100 kHz with an amplitude of 60-320 µm.

2. Use according to Claim 1 in which the gas is at a concentration which is higher than the concentration in a solution which is in equilibrium with air at 25°C and 98.1 kP (1 atm) pressure.

3. Use according to Claim 1 or claim 2 in which the surgery is lipoplasty.

4. Use according to any of Claims 1 to 3, wherein said gas is carbon dioxide.

5. Use according to any of Claims 1 to 4, wherein the gas is passed through the solution at a flow rate of from 10 to 500 ml/min.

6. Use according to Claim 5, wherein the flow rate is from 50 to 200 ml/min.

7. Use according to Claim 6, wherein the flow rate is approximately 100 ml/min.

8. Use according to any of Claims 1 to 7, wherein a water soluble scavenger is introduced into the solution prepared.

9. Use according to Claim8, wherein the water soluble scavenger is vitamin C.

## Patentansprüche

1. Verwendung eines Gases, das aus der Gruppe ausgewählt ist, die aus Kohlendioxid, Stickstoff und Gemischen daraus besteht, bei der Herstellung einer biokompatiblen, injizierbaren wäßrigen Lösung des Gases zum Gebrauch bei der hochleistungsultraschallenergieunterstützten Chirurgie mit mehr als 10 W/cm² Dauerstrichleistung bei 20-100 kHz mit einer Amplitude von 60-320 µm.

2. Verwendung nach Anspruch 1, wobei das Gas eine höhere Konzentration aufweist als die Konzentration in einer Lösung, die bei 25°C und einem Druck von 98,1 kPa (1 atm) mit Luft im Gleichgewicht ist.

3. Verwendung nach Anspruch 1 oder Anspruch 2, wobei der chirurgische Eingriff ein lipoplastischer Eingriff ist.

4. Verwendung nach einem der Ansprüche 1 bis 3, wobei das Gas Kohlendioxid ist.

5. Verwendung nach einem der Ansprüche 1 bis 4, wobei das Gas mit einer Durchflußgeschwindigkeit von 10 bis 500 ml/min durch die Lösung geleitet wird.

6. Verwendung nach Anspruch 5, wobei die Durchflußgeschwindigkeit 50 bis 200 ml/min beträgt.

7. Verwendung nach Anspruch 6, wobei die Durchflußgeschwindigkeit etwa 100 ml/min beträgt.

8. Verwendung nach einem der Ansprüche 1 bis 7, wobei ein wasserlöslicher Fänger in die hergestellte Lösung eingebracht wird.

9. Verwendung nach Anspruch 8, wobei der wasserlösliche Fänger Vitamin C ist.

## Revendications

1. Utilisation d'un gaz choisi parmi le groupe comprenant dioxyde de carbone, azote et des mélanges de ceux-ci dans une préparation d'une solution aqueuse injectable biocompatible du gaz pour une utilisation en chirurgie assistée par énergie d'ultrasons haute intensité à plus que 10 W/cm²CW à 20-100 kHz dans une amplitude de 60-320 µm.

2. Utilisation selon la revendication 1, dans laquelle le gaz est à une concentration qui est supérieure à la concentration dans une solution qui est en équilibre avec l'air à 25°C et à une pression de 98,1 kP (1 atm.)

3. Utilisation selon la revendication 1 ou 2, dans laquelle la chirurgie est une lipoplastie.

4. Utilisation selon l'une quelconque des revendications 1 à 3, dans laquelle ledit gaz est du dioxyde de carbone.

5. Utilisation selon l'une quelconque des revendications 1 à 4, dans laquelle le gaz est passé au travers de la solution à un débit d'écoulement de 10 à 500 ml/min.

6. Utilisation selon la revendication 5, dans laquelle le débit d'écoulement est de 50 à 200 ml/min.

7. Utilisation selon la revendication 6, dans laquelle le débit d'écoulement est d'approximativement 100 ml/min.

8. Utilisation selon l'une quelconque des revendications 1 à 7, dans laquelle un capteur soluble dans l'eau est introduit dans la solution préparée.

9. Utilisation selon la revendication 8, dans laquelle le capteur soluble dans l'eau est de la vitamine C.
